# EUROPEAN PATENT APPLICATION

(11) **EP 2 130 819 A2**
(43) Date of publication of application: **09.12.2009**
(21) Application number: 09157851.8
(22) Date of filing: 14.04.2009
(51) Int. Cl.: C07D 207/34, A61K 31/40

(54) **Crystalline forms of atorvastatin magnesium**

(30) Priority: 10.04.2008 IN DE09362008; 16.07.2008 IN DE16922008
(71) Applicant: Ranbaxy Laboratories Limited, Haryana 122 001 (IN)
(72) Inventor: Tyagi, Vipin, 122002 Haryana (IN); Jafri, Wajid Sajjad, 800004 Bihar (IN); Kumar, Sanjay, 250342 Uttar Pradesh (IN); Kumar, Saridi Madhava Dileep, 122101 Haryana (IN); Sathyanarayana, Swargam, 505002 Andhra Pradesh (IN); Singh, Kaptan, 201005 Uttar Pradesh (IN); Prasad, Mohan, 122002 Haryana (IN)
(74) Representative: Cronin, Brian Harold John

(57) **Abstract**

The present invention relates to crystalline forms of Atorvastatin magnesium and processes for their preparation. The crystalline forms of the present invention are designated as form X, form Y, form Z and form P of atorvastatin magnesium. The present invention further relates to pharmaceutical compositions comprising form X, form Y, form Z and form P of atorvastatin magnesium and method of using the crystalline forms.

## Description

### Field of the Invention

The present invention relates to crystalline forms of Atorvastatin magnesium and processes for their preparation. The crystalline forms of the present invention are designated as form X, form Y, form Z and form P of atorvastatin magnesium. The present invention further relates to pharmaceutical compositions comprising form X, form Y, form Z and form P of atorvastatin magnesium and method of using the crystalline forms.

### Background of the Invention

Atorvastatin is chemically [R-(R*,R*)]-2-(4-fluorophenyl)-β,δ-dihydroxy-5-(1-methylethyl)-3-phenyl-4-[(phenyl amino)carbonyl]-1H-pyrrole-1-heptanoic acid, which is an inhibitor of the enzyme, 3-hydroxy-3-methylglutaryl-coenzyme A reductase (HMG-CoA reductase) and it is useful as hypolipidemic and hypocholesterolemic agent.

The magnesium salt of atorvastatin as represented by Formula I, hereinafter referred to as atorvastatin magnesium, is disclosed in PCT Publication No. WO 2006/117761.

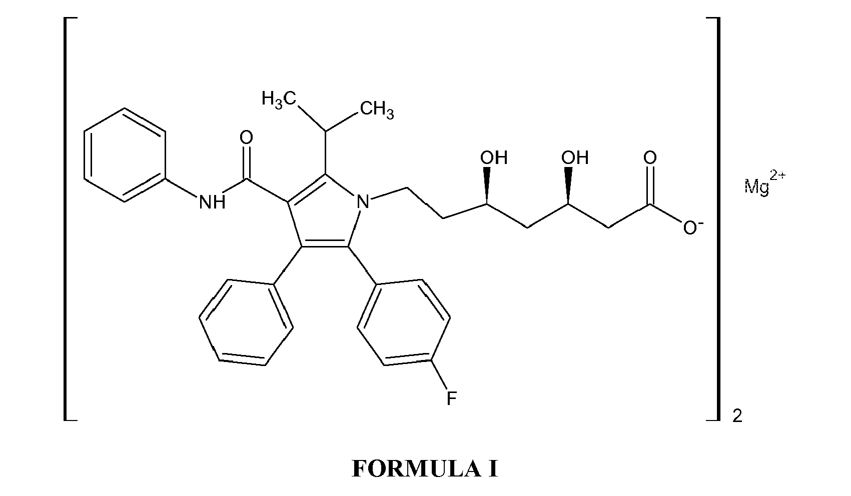

PCT Publication No. WO2006/117761 also discloses several methods for preparing atorvastatin magnesium in crystalline and amorphous forms.

The processes for preparing crystalline forms of atorvastatin magnesium are also provided in other references including PCT Publication No. WO 2007/057755, which describes processes for preparing crystalline forms A, B, C, D, E and F of atorvastatin magnesium, PCT Publication No. WO 2007/118873, which describes processes for preparing crystalline forms crystalline forms I to IX and amorphous form of atorvastatin magnesium, PCT Publication No. WO 2007/132472, which describes a process for preparing crystalline form B4 of atorvastatin magnesium, PCT Publication No. WO 2007/063551, which describes processes for preparing crystalline forms B1 and B2, and amorphous form of atorvastatin magnesium, and PCT Publication No. WO 2007/099552, which describes processes for preparing crystalline form A and isopropanol solvate of atorvastatin magnesium.

### Summary of the Invention

The present inventors have prepared crystalline forms of atorvastatin magnesium, which are designated as form X, form Y, form Z and form P. The crystalline forms of the present invention are reproducible and stable enough to develop pharmaceutical dosage forms.

### Brief Description of the Drawings

Figure 1 depicts X-Ray Powder Diffractogram (XRPD) of crystalline atorvastatin magnesium obtained according to Example 1.
Figure 2 depicts X-Ray Powder Diffractogram (XRPD) of crystalline atorvastatin magnesium obtained according to Example 2 (24 h).
Figure 3 depicts X-Ray Powder Diffractogram (XRPD) of crystalline atorvastatin magnesium obtained according to Example 2 (11 days).
Figure 3A depicts Fourier-Transform Infra-red (FTIR) spectrum of crystalline atorvastatin magnesium obtained according to Example 2 (11 days).
Figure 4 depicts X-Ray Powder Diffractogram (XRPD) of crystalline atorvastatin magnesium obtained according to Example 3.
Figure 5 depicts X-Ray Powder Diffractogram (XRPD) of crystalline atorvastatin magnesium obtained according to Example 4.
Figure 6 depicts X-Ray Powder Diffractogram (XRPD) of crystalline atorvastatin magnesium obtained according to Example 5.
Figure 7 depicts X-Ray Powder Diffractogram (XRPD) of crystalline atorvastatin magnesium obtained according to Example 6.
Figure 8 depicts X-Ray Powder Diffractogram (XRPD) of crystalline atorvastatin magnesium obtained according to Example 7.
Figure 9 depicts X-Ray Powder Diffractogram (XRPD) of crystalline atorvastatin magnesium obtained according to Example 7.
Figure 10 depicts X-Ray Powder Diffractogram (XRPD) of crystalline atorvastatin magnesium obtained according to Example 8.
Figure 11 depicts X-Ray Powder Diffractogram (XRPD) of crystalline atorvastatin magnesium obtained according to Example 9.

### Detailed Description of the Invention

A first aspect of the present invention provides crystalline form X of atorvastatin magnesium. The crystalline form X is characterized by an XRPD pattern having 2θ values substantially at 8.09 ± 0.2, 9.24 ± 0.2, 18.11 ± 0.2, 19.08 ± 0.2 and 21.59 ± 0.2. The crystalline form may be further characterized by 2θ values substantially at 3.97 ± 0.2, 5.22 ± 0.2, 17.44 ± 0.2, 19.80 ± 0.2, 20.49 ± 0.2, 20.90 + 0.2, 22.52 ± 0.2, 22.96 ± 0.2, 23.81 ± 0.2 and 25.06 ± 0.2 in some embodiments.

A second aspect of the present invention provides crystalline form Y of atorvastatin magnesium. The crystalline Form Y is characterized by an XRPD pattern having 2θ values substantially at 8.00± 0.2, 19.07 ± 0.2, 19.46 ± 0.2, 20.27 ± 0.2 and 22.51 ± 0.2. The crystalline form may be further characterized by 2θ values substantially at 9.26 ± 0.2, 18.49 ± 0.2, 20.77 ± 0.2, 23.17 ± 0.2, 24.34 ± 0.2 and 25.03 ± 0.2 in some embodiments.

A third aspect of the present invention provides crystalline form Z of atorvastatin magnesium. The crystalline Form Z of atorvastatin magnesium is characterized by an XRPD pattern having 2θ values substantially at 8.43 ± 0.2, 18.23 ± 0.2 and 18.89 ± 0.2. The crystalline form may be further characterized by 2θ values substantially at 12.30 ± 0.2 , 15.96 ± 0.2, 23.01 ± 0.2 and 25.08 ± 0.2 in some embodiments.

A fourth aspect of the present invention provides crystalline form P of atorvastatin magnesium. The crystalline Form P of atorvastatin magnesium exhibits good crystallinity. The crystalline Form P is characterized by an XRPD pattern having 2θ values substantially at 3.49 ± 0.2, 4.35 ± 0.2, 7.65± 0.2, 9.86 ± 0.2, 19.05± 0.2, 19.59± 0.2 and 20.33± 0.2. The crystalline form may be further characterized by 2θ values substantially at 16.36 ± 0.2, 18.60 ± 0.2, 21.46 ± 0.2, 22.60 ± 0.2, 25.85 ± 0.2, 26.72 ± 0.2 in some embodiments.

A fifth aspect of the present invention provides a process for the preparation of crystalline form X of atorvastatin magnesium, wherein the process comprises
a) contacting atorvastatin with a magnesium source in the presence of a solvent,
b) stirring the mixture of step a) at about 70°C or above, and
c) isolating crystalline form X of atorvastatin magnesium from the mixture thereof.

The atorvastatin used as the starting material may be obtained directly from a reaction mixture in which atorvastatin is formed, for example, by treating atorvastatin sodium with an acid, and used without isolation. The magnesium source may be, for example, magnesium hydroxide, magnesium chloride, magnesium carbonate, magnesium acetate or their hydrates. The solvent may be water or an organic solvent, or a mixture thereof. The organic solvent may be an alkanol, for example, methanol, ethanol, n-butanol, t-butanol, n-propanol, isopropanol or mixtures thereof. The mixture of atorvastatin and magnesium source in the solvent may be optionally seeded with crystalline atorvastatin magnesium. The seed may be prepared by the methods described in the prior art or by the methods disclosed in the present application. The mixture of atorvastatin and magnesium source in the solvent, optionally containing a seed, is stirred at a temperature of about 70°C or above, for example, at about 75°C to about 85°C. The stirring is carried out for a sufficient time to complete the formation of crystalline form X. The stirring may be carried out, for example, for about 1 hour to about 12 hours. The crystalline form X may be isolated from the mixture by concentration, cooling, filtration, centrifugation, decantation, drying or a combination thereof.

A sixth aspect of the present invention provides a process for the preparation of crystalline form X of atorvastatin magnesium, wherein the process comprises
a) contacting atorvastatin magnesium substantially free of other salts and impurities with an alkanol and water,
b) stirring the mixture of step a) at a temperature up to about 65°C, and
c) isolating crystalline form X of atorvastatin magnesium from the mixture thereof.

The atorvastatin magnesium used as the starting material is substantially free of other salts and impurities, such as inorganic magnesium salts and may exist in any solid form, or it may be directly from a reaction mixture in which atorvastatin magnesium is formed in solid state. The atorvastatin magnesium is contacted with alkanol and water in optional order of succession, or with a mixture of alkanol and water. The alkanol may be, for example, methanol, ethanol, n-butanol, t-butanol, n-propanol, isopropanol or mixtures thereof. The quantity of water may be more than that of alkanol. For example, water may be used about five times or more than five times to the quantity of alkanol. The mixture of atorvastatin magnesium with the alkanol and water is stirred at a temperature of up to about 65°C, for example, at about 40°C to about 65°C. The stirring is carried out for a sufficient time to complete the formation crystalline form X. The stirring may be carried out, for example, for about 1 hour to about 15 days. After stirring the mixture of atorvastatin magnesium with the alkanol and water at a temperature of up to about 65°C for a sufficient time, the mixture may be optionally further stirred at about 70°C or above, for example, at about 75°C to about 85°C. The stirring may be carried out, for example, for about 1 hour to about 15 days. The crystalline form X may be isolated from the mixture by concentration, cooling, filtration, centrifugation, decantation, drying or a combination thereof.

A seventh aspect of the present invention provides a process for the preparation of crystalline form Y of atorvastatin magnesium, wherein the process comprises
a) contacting atorvastatin with a magnesium source in the presence of a solvent,
b) stirring the mixture of step a) at a temperature of about 40°C to about 65°C, and
c) isolating crystalline form Y of atorvastatin magnesium from the mixture thereof.

The atorvastatin used as the starting material may be obtained directly from a reaction mixture in which atorvastatin is formed, for example, by treating atorvastatin sodium with an acid, and used without isolation. The magnesium source may be, for example, magnesium hydroxide, magnesium chloride, magnesium carbonate, magnesium acetate or their hydrates. The solvent may be water or an organic solvent, or a mixture thereof. The organic solvent may be an alkanol, for example, methanol, ethanol, n-butanol, t-butanol, n-propanol, isopropanol or mixtures thereof. The mixture of atorvastatin and magnesium source in the solvent may be optionally seeded with crystalline atorvastatin magnesium. The seed may be prepared by the methods described in the prior art or by the methods disclosed in the present application. The mixture of atorvastatin and magnesium source in the solvent, optionally containing a seed, is stirred at a temperature of about 40°C to about 65°C, for example, at about 50°C. The stirring is carried out for a sufficient time to complete the formation crystalline form Y. The stirring may be carried out, for example, for about 1 hour to about 1 day. The crystalline form Y may be isolated from the mixture by concentration, cooling, filtration, centrifugation, decantation, drying or a combination thereof.

An eighth aspect of the present invention provides a process for the preparation of crystalline form Y of atorvastatin magnesium, wherein the process comprises
a) contacting atorvastatin magnesium substantially free of other salts and impurities with an alkanol and water,
b) stirring the mixture of step a) at about 70°C or above, and
c) isolating crystalline form of atorvastatin magnesium from the mixture thereof.

The atorvastatin magnesium used as the starting material is substantially free of other salts and impurities, such as inorgainic magnesium salts and may exist in any crystalline or amorphous form or it may be obtained from a reaction mixture in which atorvastatin magnesium is formed and used with or without isolation in solid state. The atorvastatin magnesium is contacted with alkanol and water in optional order of succession, or with a mixture of alkanol and water. The alkanol may be, for example, methanol, ethanol, n-butanol, t-butanol, n-propanol, isopropanol or mixtures thereof. The quantity of water may be more than that of alkanol. For example, water may be used about five times or more than five times to the quantity of alkanol. The mixture of atorvastatin magnesium with the alkanol and water is stirred at a temperature of about 70°C or above, for example, at about 75°C to about 85°C. The stirring is carried out for a sufficient time to complete the formation crystalline form Y. The stirring may be carried out, for example, for about 1 hour to about 15 days. The crystalline form Y may be isolated from the mixture by concentration, cooling, filtration, centrifugation, decantation, drying or a combination thereof.

A ninth aspect of the present invention provides a process for the preparation of crystalline form Z of atorvastatin magnesium, wherein the process comprises
a) contacting atorvastatin with a magnesium source in the presence of a solvent,
b) stirring the mixture of step a) at a temperature of up to about 35°C, and
c) isolating crystalline form Z of atorvastatin magnesium from the mixture thereof.

The atorvastatin used as the starting material may exist in any solid form or it may be obtained directly from a reaction mixture in which atorvastatin is formed, for example, by treating atorvastatin sodium with an acid, and used with or without isolation. The magnesium source may be, for example, magnesium hydroxide, magnesium chloride, magnesium carbonate, magnesium acetate or their hydrates. The solvent may be water or an organic solvent, or a mixture thereof. The organic solvent may be an alkanol, for example, methanol, ethanol, n-butanol, t-butanol, n-propanol, isopropanol or mixtures thereof. Seeding with a crystalline atorvastatin magnesium may not be required for the preparation of crystalline form Z. The mixture of atorvastatin and magnesium source in the solvent is stirred at a temperature of up to about 35°C, for example, at about 15° to about 30°C. The stirring is carried out for a sufficient time to complete the formation crystalline form Z. The stirring may be carried out, for example, for about 30 minutes to about 1 day. The crystalline form Z may be isolated from the mixture by concentration, cooling, filtration, centrifugation, decantation, drying or a combination thereof.

A tenth aspect of the present invention provides a process for the preparation of crystalline form P of atorvastatin magnesium, wherein the process comprises
a) dissolving atorvastatin magnesium in an organic solvent,
b) treating the solution of step a) with an antisolvent to obtain a solid,
c) stirring the solid obtained in step b) with an alkanol and water at a temperature of about 70°C or above, and
d) isolating crystalline form P of atorvastatin magnesium from the mixture thereof.

The atorvastatin magnesium used as the starting material may exist in any crystalline or amorphous form or it may be obtained directly from a reaction mixture in which atorvastatin magnesium is formed and used with or without isolation. The atorvastatin magnesium is dissolved in an organic solvent. The organic solvent may be an alkanol, for example, methanol, ethanol, n-butanol, t-butanol, n-propanol or isopropanol, or an ether, for example, tetrahydrofuran, or a mixture thereof. The dissolution may be effected by heating to about 35°C or above, for example, to about 35°C to about 45°C. The solution so obtained may optionally be filtered to remove any undissolved material. The solution so obtained is treated with an antisolvent, for example, water. The antisolvent may be added drop-wise for about 30 minutes to about 10 hours. The mixture so obtained may be optionally stirred to facilitate the precipitation of solid. The solid so obtained may be isolated by filtration. The solid is contacted with alkanol and water in optional order of succession, or with a mixture of alkanol and water. The alkanol may be, for example, methanol, ethanol, n-butanol, t-butanol, n-propanol, isopropanol or mixtures thereof. The quantity of water may be more than that of alkanol. For example, water may be used about five times or more than five times to the quantity of alkanol. The mixture so obtained is stirred at a temperature of about 70°C or above, for example, at about 75°C to about 85°C. The stirring is carried out for a sufficient time to complete the formation crystalline form P. The stirring may be carried out, for example, for about 15 hours to about 15 days. The crystalline form P may be isolated from the mixture by concentration, cooling, filtration, centrifugation, decantation, drying or a combination thereof.

An eleventh aspect of the present invention provides a pharmaceutical composition comprising the crystalline form X of atorvastatin magnesium and a pharmaceutically acceptable excipient, diluent and/or carrier.

A twelfth aspect of the present invention provides a method for treating or preventing hyperlipidemia, hypercholesterolemia, Alzheimer's disease, atherosclerosis, xanthoma and in synergism with other drugs for treatment of phytosterolemia lipase deficiency and the like, which comprises administering to a mammal, in need thereof, an effective amount of crystalline form X of atorvastatin magnesium.

A thirteenth aspect of the present invention provides a pharmaceutical composition comprising the crystalline form Y of atorvastatin magnesium and a pharmaceutically acceptable excipient, diluent and/or carrier.

A fourteenth aspect of the present invention provides a method for treating or preventing hyperlipidemia, hypercholesterolemia, Alzheimer's disease, atherosclerosis, xanthoma and in synergism with other drugs for treatment of phytosterolemia lipase deficiency and the like, which comprises administering to a mammal, in need thereof, an effective amount of crystalline form Y of atorvastatin magnesium.

A fifteenth aspect of the present invention provides a pharmaceutical composition comprising the crystalline form Z of atorvastatin magnesium and a pharmaceutically acceptable excipient, diluent and/or carrier.

A sixteenth aspect of the present invention provides a method for treating or preventing hyperlipidemia, hypercholesterolemia, Alzheimer's disease, atherosclerosis, xanthoma and in synergism with other drugs for treatment of phytosterolemia lipase deficiency and the like, which comprises administering to a mammal, in need thereof, an effective amount of crystalline form Z of atorvastatin magnesium.

A seventeenth aspect of the present invention provides a pharmaceutical composition comprising the crystalline form P of atorvastatin magnesium and a pharmaceutically acceptable excipient, diluent and/or carrier.

An eighteenth aspect of the present invention provides a method for treating or preventing hyperlipidemia, hypercholesterolemia, Alzheimer's disease, atherosclerosis, xanthoma and in synergism with other drugs for treatment of phytosterolemia lipase deficiency and the like, which comprises administering to a mammal, in need thereof, an effective amount of crystalline form P of atorvastatin magnesium.

While the present invention has been described in terms of its specific embodiments, certain modifications and equivalents will be apparent to those skilled in the art and are intended to be included within the scope of the present invention.

### EXAMPLES

### Example 1: Preparation of Form Z of Atorvastatin Magnesium

### Stage I: Preparation of Atorvastatin

Atorvastatin calcium (100 g) and de-ionized water (1500 ml) were stirred for 10 minutes at room temperature and pH of the slurry was adjusted to 3.5 by drop-wise addition of 1 N hydrochloric acid. The mixture was stirred for 10 minutes at room temperature and ethyl acetate (1000 ml) was added to the mixture. The mixture was further stirred for 10 minutes and the pH of the solution was adjusted to 5.5 by the addition of hydrochloric acid. After stirring of 10 minutes, a clear solution was obtained. The organic layer was separated and the aqueous layer was extracted with ethyl acetate (500 ml). The combined layer was washed with 1N hydrochloric acid (100 ml) followed by aqueous sodium chloride solution (20 %, 300 ml). The organic layer was concentrated completely under reduced pressure at 30°C. Methanol (2 X 100 ml) was added to the concentrated mass and recovered completely under reduced pressure to obtain the title compound as a residue.

### Stage II: Preparation of Form Z of Atorvastatin Magnesium

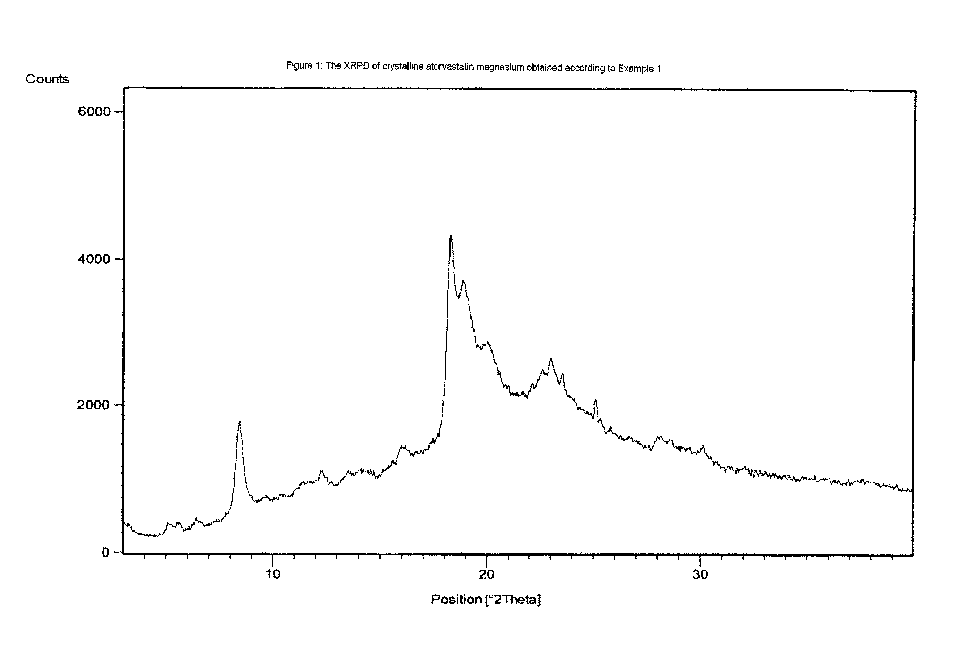

The residue of Stage-I was dissolved in methanol (1000 ml) at room temperature and stirred for 10 minutes. A solution of sodium hydroxide (16 g sodium hydroxide dissolved in 800 ml de-ionized water) was added drop-wise in 30 minutes duration to the solution obtained above at room temperature and stirred for 4 hours. The solution was washed with toluene (2 X 100 ml) and filtered through Celite bed. The Celite bed was washed with methanol (250 ml). The pH of the combined solution was slowly adjusted to 7.9 by the addition of 1N hydrochloric acid. The temperature of the solution was slowly raised to 50°C. An aqueous solution of magnesium acetate tetrahydrate (20 g dissolved in 2.4 L of de-ionized water) was added in 1 hour at 50°C to the solution obtained above. The mixture was slowly cooled 25°C and stirred for 1 hour at 25° to 30°C, filtered, washed with de-ionized water (250 ml) to obtain a wet material (200 g). The wet material (10 g) was dried under reduced pressure at 40° to 45°C till the moisture content attained the range of 3 to 5% to obtain the title compound (3.5 g) having XRPD pattern as depicted in Figure 1.

### Example 2: Preparation of Form X of Atorvastatin Magnesium

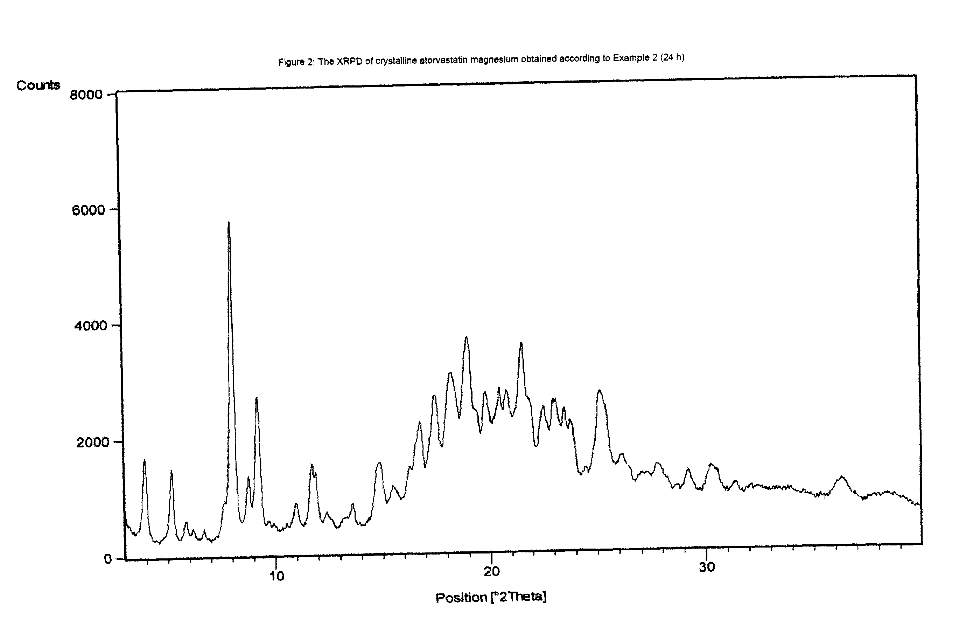

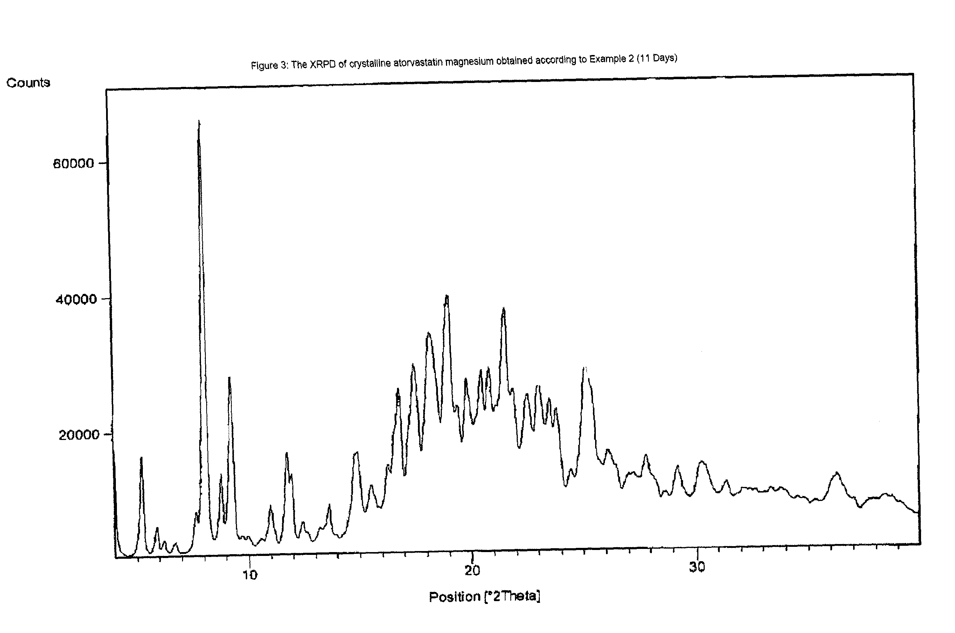

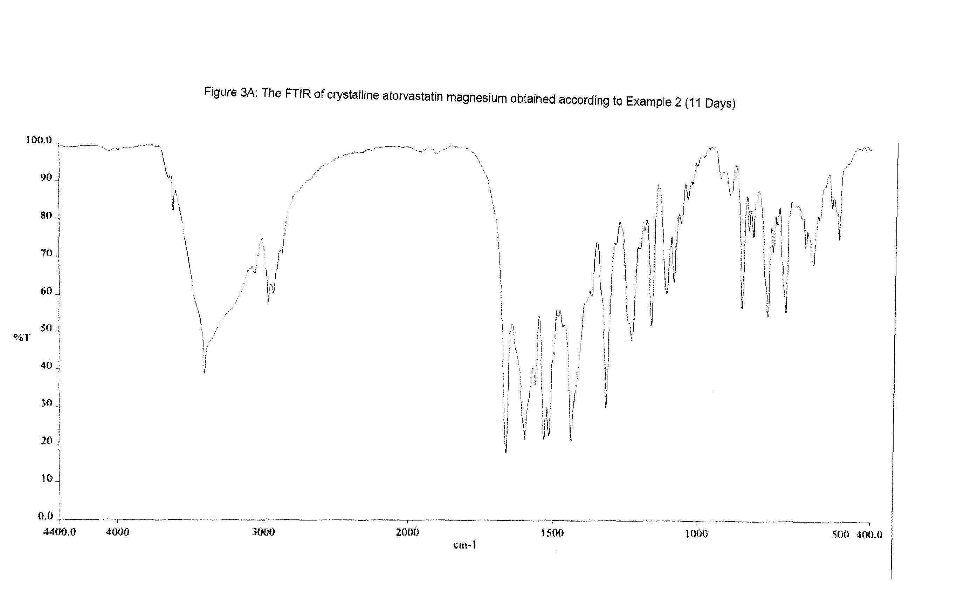

The residue (atorvastatin) as obtained by the method of Stage-I of Example 1 was dissolved in methanol (1000 ml) at room temperature and stirred for 10 minutes. A solution of sodium hydroxide (16 g of sodium hydroxide dissolved in 800 ml of de-ionized water) was added drop-wise in 30 minutes duration to the solution obtained above at room temperature and stirred for 4 hours. The solution was washed with toluene (2 X 100 ml) and filtered through Celite bed. The Celite bed was washed with methanol (250 ml). The pH of the combined solution was slowly adjusted to 7.9 by the addition of 1N hydrochloric acid. The temperature of the solution was slowly raised to 50°C. An aqueous solution of magnesium acetate tetrahydrate (20 g dissolved in 2.4 L of de-ionized water) was added in 1 hour at 50°C to the solution obtained above. The mixture was slowly cooled 25°C and stirred for 1 hour at 25° to 30°C, filtered, washed with de-ionized water (250 ml) to obtain a wet material. The wet material was mixed with aqueous methanol (de-ionized water - 1700 ml; methanol - 300 ml) at room temperature. The temperature was raised to 65°C and stirred for 15 hours at 60° to 65°C. The temperature of the reaction mixture was further raised to 75° to 78°C and stirred at 75° to 78°C for 11 days (The XRPD of the solid formed after 24 hours was determined and the XRPD is depicted in Figure 2). The solid was filtered after 11 days, washed with de-ionized water (200 ml), dried under vacuum at 45° to 50°C till the moisture content attained the range of 3 to 5% to obtain the title compound (68 g) having XRPD pattern as depicted in Figure 3 and FTIR pattern as depicted in Figure 3A.

### Example 3: Preparation of Form Y of Atorvastatin Magnesium

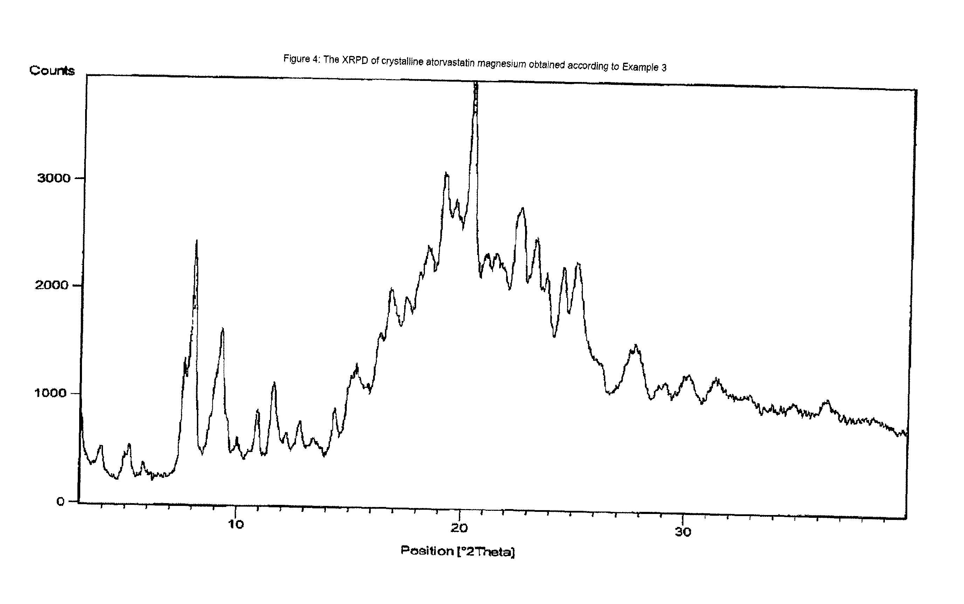

The residue (atorvastatin) as obtained by the method of Stage-I of Example 1 was dissolved in methanol (1000 ml) at room temperature and stirred for 10 minutes. A solution of sodium hydroxide (16 g of sodium hydroxide dissolved in 800 ml of de-ionized water) was added to the reaction mixture in 30 minutes duration at room temperature and stirred for 4 hours. The solution was washed with toluene (2 X 250 ml). The pH of the solution was slowly adjusted to 8.0 by the addition of 1N hydrochloric acid and filtered through Celite bed. The Celite bed was washed with methanol (250 ml). The temperature of the combined solution was slowly raised to 50°C. An aqueous solution of magnesium acetate tetrahydrate (20 g dissolved in 2.4 L of de-ionized water) was added in 1 hour at 50°C to the solution so obtained. After the addition of 400 ml of aqueous magnesium acetate, the mixture was seeded with crystalline form X of atorvastatin magnesium (5g) and the addition of remaining magnesium acetate solution was subsequently completed. The mixture was stirred for 15 hours at 50°C, filtered and washed with de-ionized water (200 ml) to obtain a wet material (200 g). The wet material (10 g) was dried under vacuum at 50° to 55°C till the moisture content attained the range of 3 to 5% to obtain the title compound (3.4 g) having XRPD pattern as depicted in Figure 4.

### Example 4: Preparation of Form X of Atorvastatin Magnesium

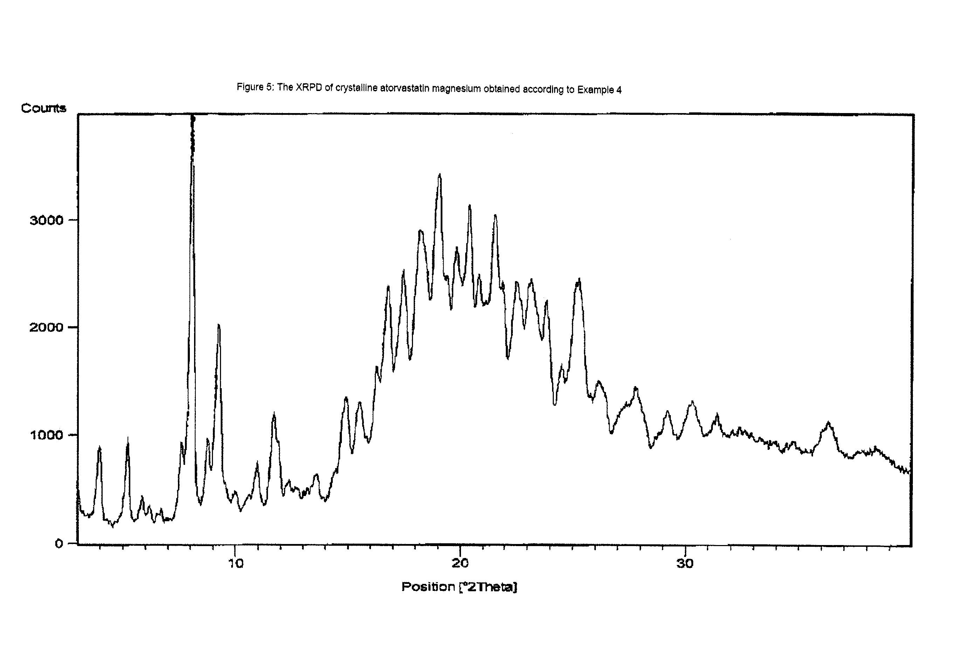

The residue (atorvastatin) as obtained by the method of Stage-I of Example 1 was dissolved in methanol (1000 ml) at room temperature and stirred for 10 minutes. A solution of sodium hydroxide (16 g of sodium hydroxide dissolved in 800 ml of de-ionized water) was added to the reaction mixture in 30 minutes duration at room temperature and stirred for 4 hours. The solution was washed with toluene (2 X 250 ml). The pH of the solution was slowly adjusted to 8.0 by the addition of 1N hydrochloric acid and filtered through Celite bed. The Celite bed was washed with methanol (250 ml). The temperature of the combined solution was slowly raised to 50°C. An aqueous solution of magnesium acetate tetrahydrate (20 g dissolved in 2.4 L of de-ionized water) was added in 1 h at 50°C to the solution so obtained. After the addition of 400 ml of aqueous magnesium acetate, the mixture was seeded with crystalline form X of atorvastatin magnesium (5g) and the addition of remaining magnesium acetate solution was subsequently completed. The mixture was stirred for 15 hours at 50°C, filtered and washed with de-ionized water (200 ml) to obtain a wet material. The wet material was mixed with aqueous methanol (de-ionized water - 1700 ml; methanol - 300 ml) at room temperature. The temperature of the mixture was slowly raised to 50°C, stirred for 7 days at 50°C, filtered, washed with de-ionized water (200 ml) and dried under reduced pressure at 50° to 55°C till the moisture content attained the range of 3 to 5% to obtain the title compound (80 g) having XRPD pattern as depicted in Figure 5.

### Example 5: Preparation of Form X of Atorvastatin Magnesium

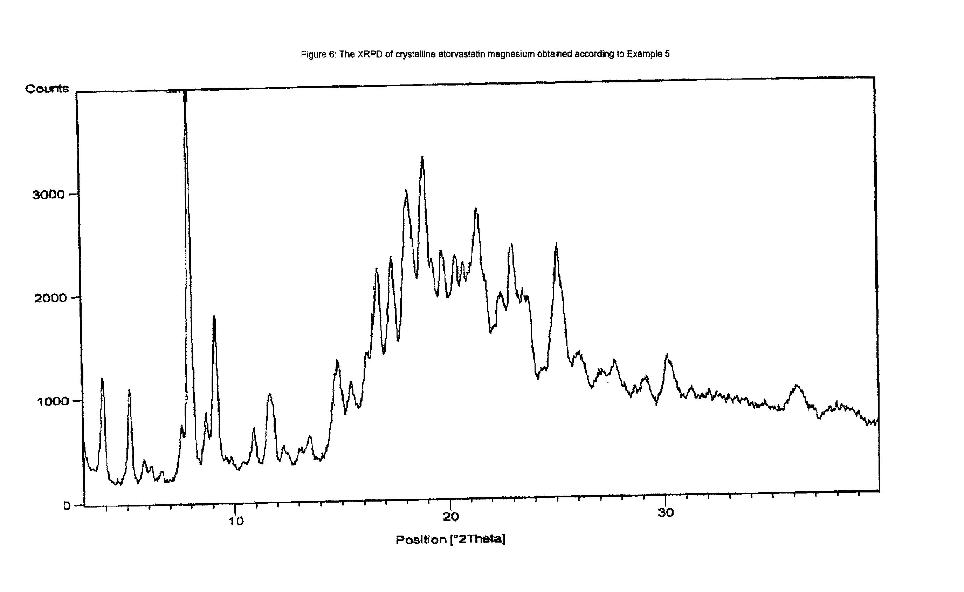

The residue (atorvastatin) as obtained by the method of Stage-I of Example 1 was dissolved in methanol (1000 ml) at room temperature and stirred for 10 minutes. An aqueous solution of sodium hydroxide (16 g of sodium hydroxide dissolved in 800 ml of de-ionized water) was added in 30 minutes duration at room temperature to the solution so obtained and stirred for 4 hours. The solution was washed with toluene (2 X 250 ml), filtered through Celite bed and the bed was washed with methanol (250 ml). The pH of the solution was adjusted to 7.9 by slow addition of 1N hydrochloric acid. The temperature of the solution was slowly raised to 70° to 75°C. An aqueous solution of magnesium acetate tetrahydrate (20 g dissolved in 2.4 L of de-ionized water) was added to the solution so obtained in 1 hour at 70° to 75°C and seeded with crystalline form X of atorvastatin magnesium (5 g). The mixture was stirred for 6 hours at 75° to 80°C, cooled to 45°C, filtered and washed with de-ionized water (200 ml) to obtain a wet material (200 g). The wet material (10 g) was dried under vacuum at 50° to 55°C till the moisture content attained the range of 3 to 5% to obtain the title compound (3.3 g) having XRPD pattern as depicted in Figure 6.

### Example 6: Preparation of Form Y of Atorvastatin Magnesium

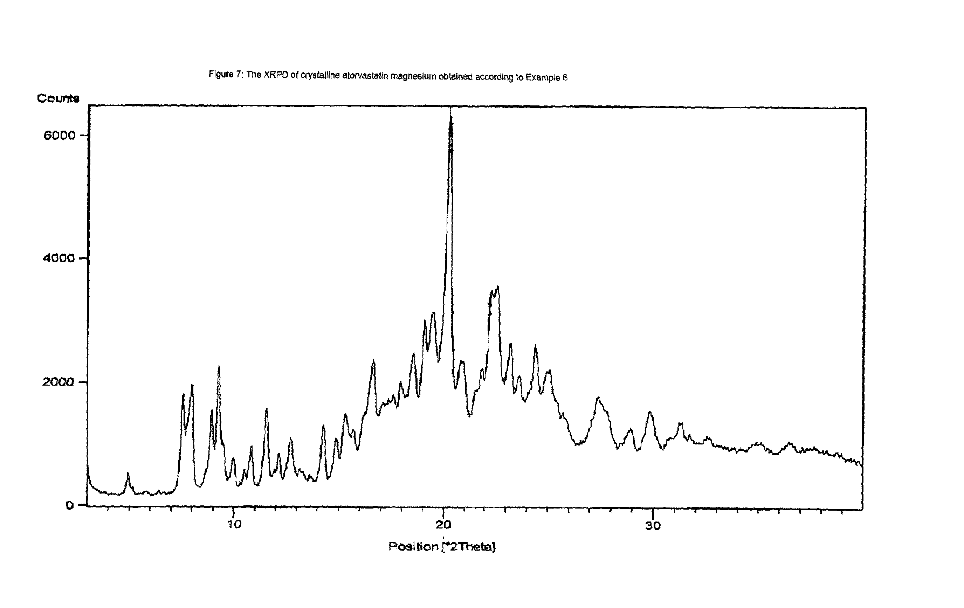

The residue (atorvastatin) as obtained by the method of Stage-I of Example 1 was dissolved in methanol (1000 ml) at room temperature and stirred for 10 minutes. An aqueous solution of sodium hydroxide (16 g of sodium hydroxide dissolved in 800 ml of de-ionized water) was added in 30 minutes duration at room temperature to the solution so obtained and stirred for 4 hours. The solution was washed with toluene (2 X 250 ml), filtered through Celite bed and the bed was washed with methanol (250 ml). The pH of the solution was adjusted to 7.9 by slow addition of 1N hydrochloric acid. The temperature of the solution was slowly raised to 70° to 75°C. An aqueous solution of magnesium acetate tetrahydrate (20 g dissolved in 2.4 L of de-ionized water) was added to the solution so obtained in 1 hour at 70° to 75°C and seeded with crystalline form X of atorvastatin magnesium (5 g). The mixture was stirred for 6 hours at 75° to 80°C, cooled to 45°C, filtered and washed with de-ionized water (200 ml) to obtain a wet material. The wet material was mixed with aqueous methanol (de-ionized water - 1700 ml; methanol - 300 ml) at room temperature. The temperature was slowly raised to 75° to 80°C, stirred for 4 days at 75° to 80°C, filtered and washed with de-ionized water (200 ml). The solid was dried under vacuum at 50° to 55°C till the moisture content attained the range of 3 to 5% to obtain the title compound (90 g) having XRPD pattern as depicted in Figure 7.

### Example 7: Preparation of Form Y of Atorvastatin Magnesium

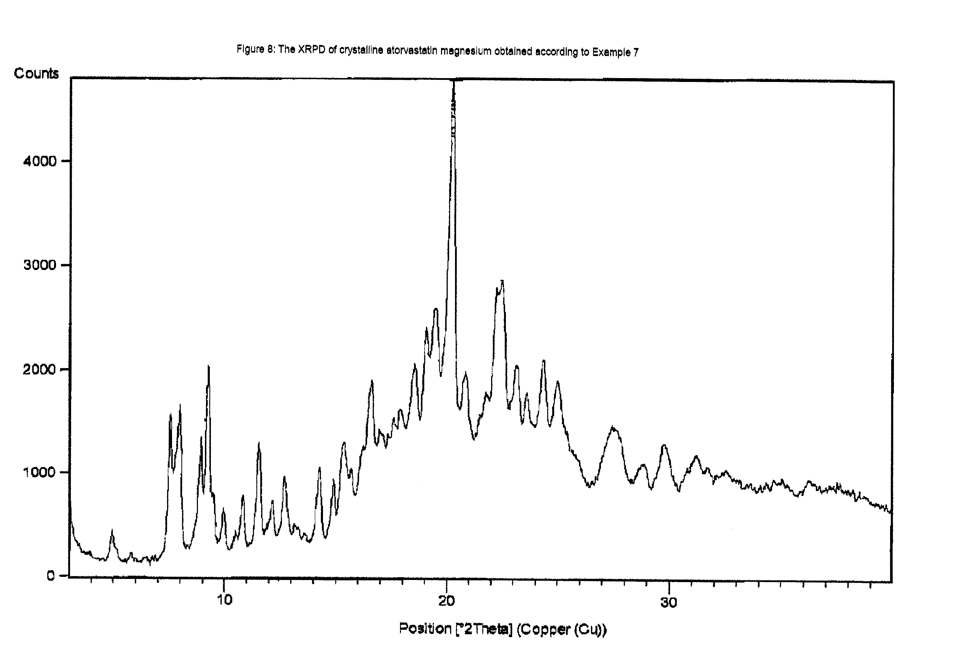

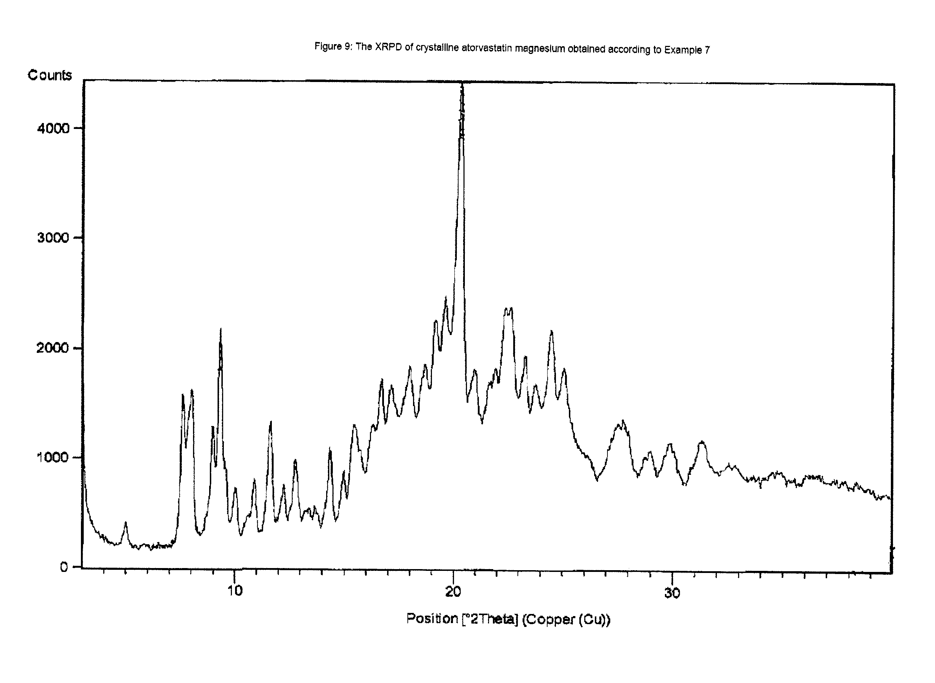

The residue (atorvastatin) as obtained by the method of Stage-I of Example 1 was dissolved in ethanol (1000 ml) at room temperature and stirred for 10 minutes. A solution of sodium hydroxide (16 g of sodium hydroxide dissolved in 800 ml of de-ionized water) was added in 30 minutes duration at room temperature to the solution so obtained and stirred for 4 hours. The pH of the solution was adjusted to 8.0 by slow addition of 1N hydrochloric acid and filtered through Celite bed. The bed was washed with ethanol (250 ml). The combined solution was washed with toluene (2 X 100 ml) and the temperature of the solution was raised to 50°C. An aqueous solution of magnesium acetate tetrahydrate (20 g dissolved in 2.4 L of de-ionized water) was added to the solution so obtained in 1 hour at 50°C and seeded with crystalline form X of atorvastatin magnesium. The mixture was stirred for 12 hours at 50°C, filtered and washed with de-ionized water (200 ml) to obtain a wet material (200 g; 10 g of the wet material was dried under vacuum at 50° to 55°C till the moisture content attained the range of 3 to 5% and the XRPD of the dried solid was determined. The XRPD is depicted in Figure 8). The wet material was mixed with aqueous ethanol (de-ionized water - 1700 ml; ethanol - 300 ml) at room temperature. The temperature was slowly raised to 75° to 80°C, stirred for 24 hours at 75° to 80°C, filtered and washed with de-ionized water (200 ml). The solid was dried under vacuum at 50° to 55°C till the moisture content attained the range of 3 to 5% to obtain the title compound (90 g) having XRPD pattern as depicted in Figure 9.

### Example 8: Preparation of Form Y of Atorvastatin Magnesium

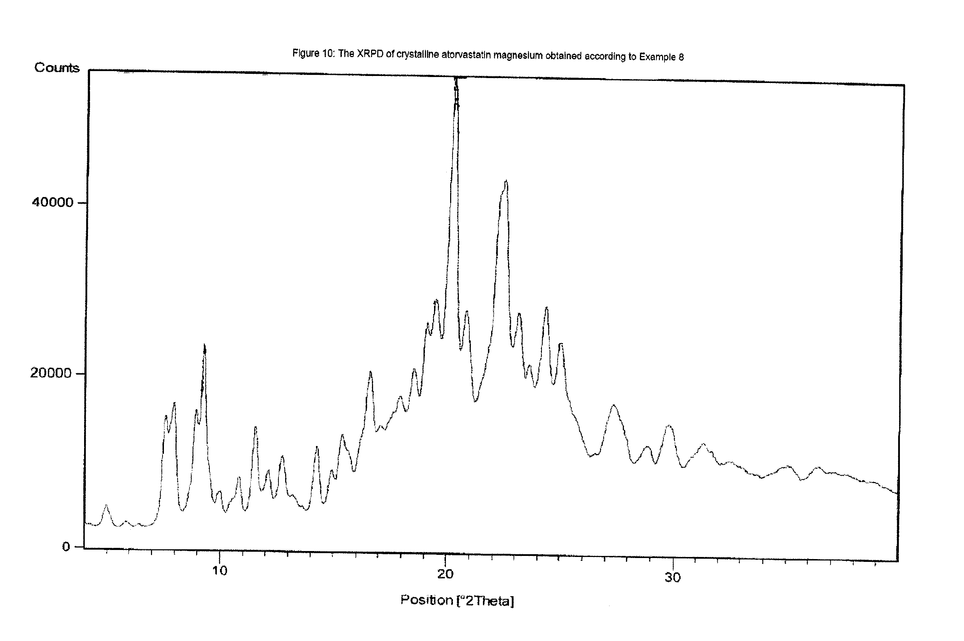

A mixture of (4R-cis)-1,1-dimethyl-6-{2-{2-(4-fluorophenyl)-5-(1-methylphenyl)-3-phenyl-4-[(phenylamino)-carbonyl]-1H-pyrrole-1-yl}-ethyl-2,2-dimethyl-1,3-dioxane-4-acetate (100 g) in methanol (2100 ml) was cooled to 20° to 25°C. The pH of the reaction mixture was adjusted to 2.0 by slow addition of 1 N hydrochloric acid in 25 to 30 minutes at 20° to 25°C. The temperature of the reaction mixture was raised to 25° to 30°C and stirred for 6 to 8 hours. After completion of the reaction, sodium hydroxide solution (23 g of sodium hydroxide dissolved in 230 ml of de-ionized water) was added in 15 to 20 minutes at 25° to 30°C till the pH attained the range of 11.8 to 12.2. The reaction mixture was stirred for 4 to 6 hours at 25° to 30°C. After the completion of the reaction, the reaction mixture was filtered through Celite bed and the bed was washed with methanol (100 ml). The filtrate (volume - 2700 ml) was concentrated under reduced pressure at 45° to 50°C to a residual volume of 600 to 700 ml. De-ionized water (900 ml), methanol (190 ml) and methyl tertiarybutyl ether (400 ml) were added to concentrate at 40°C. The reaction mixture was cooled and stirred for 15 minutes at 25° to 30°C. The layers were separated and the aqueous layer was extracted with methyl tertiarybutyl ether (300 ml) at 25° to 30°C. The pH of the aqueous layer was adjusted to 1.8 to 2.2 using 2N hydrochloric acid. The reaction mixture was stirred for 30 minutes at 25° to 30°C and methyl tertiarybutyl ether (400 ml) was added. The mixture was stirred for 10 to 15 minutes and the layers were separated. The organic layer was washed with de-ionized water (3 x 150 ml) at 25° to 30°C till the pH became neutral. Methanol (260 ml) and sodium hydroxide (8.0 g dissolved in 1200 ml of de-ionized water) were added to the organic layer and the mixture was stirred for 30 minutes at 25° to 30°C. The layers were separated and the aqueous layer was extracted with methyl tertiarybutyl ether (300 ml) at 25° to 30°C. The aqueous layer was filtered over Celite bed and washed with de-ionized water (100 ml). Tetrahydrofuran (400 ml) was added to the aqueous layer and the temperature was raised to 48° to 52°C. A solution of magnesium acetate tetrahydrate (25.0 g dissolved in 560 ml of De-ionized water) was added to the reaction mixture in 60 to 90 minutes. After the addition of one-fourth of magnesium acetate solution, crystalline form Y of atorvastatin magnesium (5 g) was added at 48° to 52°C and the remaining part of magnesium acetate solution was added. The mixture was stirred for 1 h at 50° to 55°C. The temperature of the reaction mixture was slowly raised to 58° to 65°C with downward distillation to remove the residual methyl tertiarybutyl ether and maintained for 1 hour. The reaction mixture was stirred at 58° to 65°C for 24 hours. The reaction mixture was cooled slowly to 30° to 35°C in 2 hours and maintained at 30° to 35°C for 1 hour. The solid was filtered and washed with a solution of water (900 ml) and methanol (125 ml) followed by de-ionized water (3 x 200 ml). The solid was dried under reduced pressure for 12 to 14 hours at 40° to 45°C. The solid was mixed with t-butanol (300 ml) and de-ionized water (1700 ml) at 25° to 30°C under stirring. The mixture was stirred at 74° to 76°C for 96 hours. The solid was filtered and subjected to suction for 1 hour. The solid was washed with a mixture of de-ionized water and t-butanol (100 ml; 85:15) and dried under reduced pressure at 40° to 45°C for 12 hours to obtain the title compound (90 g) having XRPD pattern as depicted in Figure 10.

### Example 9: Preparation of Form P of Atorvastatin Magnesium

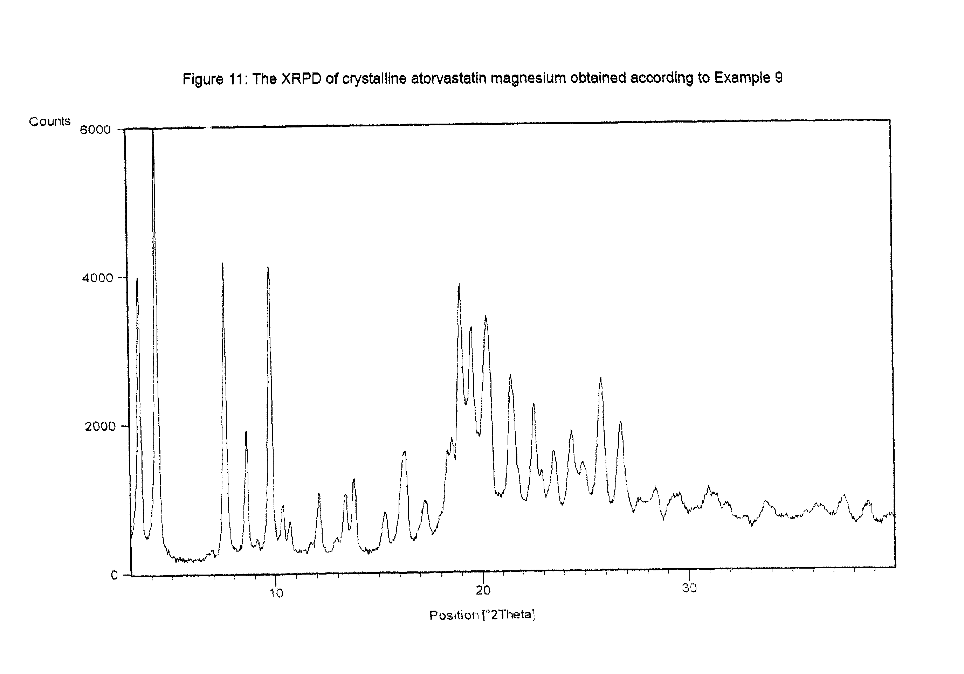

Methanol (600 ml) and tetrahydrofuran (600 ml) were added to atorvastatin magnesium (100 g) at room temperature. The temperature of the reaction mixture was raised to 38° to 42°C and maintained for 30 to 45 minutes. The reaction mixture was then filtered through Celite bed and the bed was washed with methanol (100 ml). De-ionized water (2 L) was added drop-wise to the filtrate in 90 to 120 minutes at 30° to 32°C and the mixture was stirred for 5 to 7 hours at 30° to 32°C. The solid was filtered, washed with de-ionized water (100 ml) and dried under reduced pressure at 50° to 55°C for 12 to 14 hours. The solid obtained (100 g) was added to t-butanol (300 ml) and de-ionized water (1700 ml) at 25° to 30°C under stirring. The mixture was stirred at 78° to 82°C for 96 hours. The mixture was filtered and subjected to suction for 1 h. The solid was washed with a mixture of de-ionized water and t-butanol (100 ml; 85:15) and dried under reduced pressure at 50° to 55°C for 12 hours to obtain the title compound (83 g) having XRPD pattern as depicted in Figure 11.

## Claims

1. Crystalline form X of atorvastatin magnesium **characterized by** an XRPD pattern having 2θ values substantially at 8.09 ± 0.2, 9.24 ± 0.2, 18.11± 0.2, 19.08 ± 0.2 and 21.59 ± 0.2.

2. Crystalline form X of atorvastatin magnesium according to claim 1 further **characterized by** 2θ values substantially at 3.97 ± 0.2, 5.22 ± 0.2, 8.09 ± 0.2, 9.24 ± 0.2, 17.44 ± 0.2, 18.11± 0.2, 19.08 ± 0.2, 19.80 ± 0.2, 20.49 ± 0.2, 20.90 ± 0.2, 21.59 ± 0.2, 22.52 ± 0.2, 22.96 ± 0.2, 23.81 ± 0.2 and 25.06 ± 0.2.

3. Crystalline form Y of atorvastatin magnesium **characterized by** an XRPD pattern having 2θ values substantially at 8.00± 0.2, 19.07 ± 0.2, 19.46 ± 0.2, 20.27 ± 0.2 and 22.51 ± 0.2.

4. Crystalline form Y of atorvastatin magnesium according to claim 3 further **characterized by** 2θ values substantially at 8.00 ± 0.2, 9.26 ± 0.2, 18.49 ± 0.2, 19.07 ± 0.2, 19.46 ± 0.2, 20.27 ± 0.2, 20.77 ± 0.2, 22.51 ± 0.2, 23.17 ± 0.2, 24.34 ± 0.2 and 25.03 ± 0.2.

5. Crystalline form Z of atorvastatin magnesium **characterized by** an XRPD pattern having 2θ values substantially at 8.43 ± 0.2, 18.23 ± 0.2 and 18.89 ± 0.2.

6. Crystalline form Z of atorvastatin magnesium according to claim 5 further **characterized by** 2θ values substantially at 8.43 ± 0.2, 12.30 ± 0.2, 15.96 ± 0.2, 18.23 ± 0.2, 18.89 ± 0.2, 23.01 ± 0.2 and 25.08 ± 0.2.

7. Crystalline form P of atorvastatin magnesium **characterized by** an XRPD pattern having 2θ values substantially at 3.49 ± 0.2, 4.35 ± 0.2, 7.65± 0.2, 9.86 ± 0.2, 19.05± 0.2, 19.59± 0.2 and 20.33± 0.2.

8. Crystalline form P of atorvastatin magnesium according to claim 7 further **characterized by** 2θ values substantially at 3.49 ± 0.2, 4.35 ± 0.2, 7.65± 0.2, 9.86 ± 0.2, 16.36 ± 0.2, 18.60 ± 0.2, 19.05± 0.2, 19.59± 0.2, 20.33± 0.2, 21.46 ± 0.2, 22.60 ± 0.2, 25.85 ± 0.2, 26.72 ± 0.2.

9. A process for the preparation of crystalline form X of atorvastatin magnesium, wherein the process comprises
a) contacting atorvastatin with a magnesium source in the presence of a solvent,
b) stirring the mixture of step a) at about 70°C or above, and
c) isolating crystalline form X of atorvastatin magnesium from the mixture thereof.

10. A process for the preparation of crystalline form X of atorvastatin magnesium, wherein the process comprises
a) contacting atorvastatin magnesium substantially free of other salts and impurities with an alkanol and water,
b) stirring the mixture of step a) at a temperature up to about 65°C, and
c) isolating crystalline form X of atorvastatin magnesium from the mixture thereof.

11. A process for the preparation of crystalline form Y of atorvastatin magnesium, wherein the process comprises
a) contacting atorvastatin with a magnesium source in the presence of a solvent,
b) stirring the mixture of step a) at a temperature of about 40°C to about 65°C, and
c) isolating crystalline form Y of atorvastatin magnesium from the mixture thereof.

12. A process for the preparation of crystalline form Y of atorvastatin magnesium, wherein the process comprises
a) contacting atorvastatin magnesium substantially free of other salts and impurities with an alkanol and water,
b) stirring the mixture of step a) at about 70°C or above, and
c) isolating crystalline form Y of atorvastatin magnesium from the mixture thereof.

13. A process for the preparation of crystalline form Z of atorvastatin magnesium, wherein the process comprises
a) contacting atorvastatin with a magnesium source in the presence of a solvent,
b) stirring the mixture of step a) at a temperature of up to about 35°C, and
c) isolating crystalline form Z of atorvastatin magnesium from the mixture thereof.

14. A process for the preparation of crystalline form P of atorvastatin magnesium, wherein the process comprises
a) dissolving atorvastatin magnesium in an organic solvent,
b) treating the solution of step a) with an antisolvent to obtain a solid,
c) stirring the solid obtained in step b) with an alkanol and water at a temperature of about 70°C or above, and
d) isolating crystalline form P of atorvastatin magnesium from the mixture thereof.

15. A pharmaceutical composition comprising one or more of crystalline forms X, Y, Z and P of atorvastatin magnesium and a pharmaceutically acceptable excipient, diluent and/or carrier.
